# EUROPEAN PATENT APPLICATION

(11) **EP 0 571 112 A2**
(43) Date of publication of application: **24.11.1993**
(21) Application number: 93303577.6
(22) Date of filing: 07.05.1993
(51) Int. Cl.: A61B 5/00

(54) **Electrochemical sensor**

(30) Priority: 22.05.1992 US 887615
(71) Applicant: BIOMEDICAL SENSORS LTD, High Wycombe, Buckinghamshire, PH13 5RE (GB)
(72) Inventor: Markle, David R., Paoli, Pennsylvania 19301 (US); Hendry, Stuart P., High Wycombe, Buckinghamshire (GB)
(74) Representative: Hayles, James Richard

(57) **Abstract**

An electrochemical sensor for the determination of the partial pressure of oxygen in a bloodstream comprising a cathode 20 and an anode 21 immersed in an electrolyte 17 contained in a chamber 23 defined by an oxygen gas permeable membrane 19 wherein the conductor 12 forming the anode 21 is folded into a "U" shape 22 so that the exposed distal end surface 13 thereof faces the distal end surface 13 of the cathode 20.

## Description

This invention relates to an electrochemical sensor. More particularly, the invention is concerned with an electrochemical sensor that prevents rapid failure of the conductors therein. The invention is also concerned with an electrical circuit comprising an electrochemical sensor and a current sensitive measuring device; and a method for manufacturing the electrochemical sensor.

United States Patent No. 3,905,888 discloses an electrochemical sensor for determining the oxygen partial pressure, particularly in biological media in vivo. The sensor has two electrodes disposed in a chamber which contains an electrolyte and the walls of which are permeable to oxygen. The electrodes comprise a pair of wires of different lengths parallel to the longitudinal axis of the chamber. The longer wire has an insulating layer in the region over which the shorter wire extends, the active surfaces of the electrodes being formed by the non-insulated surfaces of the wire ends. Typically the electrode with the longer active surface is the anode and the wire is preferably made of silver. The electrode with the shorter active surface is the anode and the wire is preferably made of platinum or, alternatively, silver.

If holes exist in the insulation of the cathode dendrites will grow through the hole to the anode and short out the sensor. To avoid that problem the length over which an insulated portion is next to an insulated part is minimized. That geometric arrangement also is prone to failure because the metal consumed tends to be at the base of the anode near the distal end of the insulation, which is the site for the shortest path for flow of current between the anode and the cathode. After a while the tip of the anode becomes separated from its insulated base when the silver thereat has been consumed.

It has now been found that a longer lived and more reliable structure for the conductors in an electrochemical sensor is provided by the novel sensor of the present invention.

In accordance with the present invention there is provided an electrochemical sensor for the determination of the partial pressure of oxygen in a blood stream comprising a cathode and an anode immersed in an electrolyte contained in a closed chamber defined by an oxygen gas permeable membrane, characterized in that each of the cathode and anode comprises the exposed uninsulated distal portion of a plurality of elongate insulated conductors each having an exposed uninsulated distal end surface and a proximal end; the elongate insulated conductors are associated with a support which positions each conductor with the insulated portions in parallel and the elongate conductor or conductors which form the anode is folded into a "U" shape so that the exposed distal end surface thereof faces the distal end surface of the cathode; the gap between the facing distal end surfaces of the cathode and anode being filled with an electrolyte to permit the flow of electric current energy across the gap; and the proximal end of each of the conductors is adapted to be connected to a current sensitive measuring device in circuit with a power source for monitoring changes in the flow of electric current between the distal end surfaces.

In a preferred embodiment of the sensor according to the invention each of the cathode and anode comprises a single conductor. Preferably the facing distal end surfaces are parallel to each other, although this is not essential.

Preferably the cathode is made from silver and the anode is made from silver or silver chloride.

The gap between the facing distal end surfaces of the elongate insulated conductors defines approximately equal distances between the facing distal end surfaces. The electrolyte, preferably a buffered potassium chloride solution, which fills the gap permits flow of electric current across the gap and between the distal end surfaces.

The invention also provides an electrical circuit for the in vivo monitoring of the partial pressure of oxygen in a patient's blood stream characterized in that it comprises, in combination, an electrochemical sensor as described above connected, through the proximal end of each of the conductors to a current sensitive measuring device in circuit with a power source, whereby changes in the flow of electrical current between the distal end surfaces of the conductors may be monitored.

The electrical circuit most preferably has two conductors in the plurality. The facing distal end surfaces are preferably substantially parallel to each other, although this is not essential. An electrochemical cell preferably results from the conductors, the electrolyte in the gap and the current sensitive measuring device in circuit with a power source. The electrochemical cell is an oxygen sensitive device generating a current flow between one conductor, that is an anode and another conductor, that is a cathode. The current is measurable at the proximal ends.

The electrolyte is contained within a gas permeable membrane that permits oxygen to diffuse therethrough and the cathode is an insulated metal, preferably silver wire and the anode is an insulated silver and/or silver chloride wire. Each wire is stripped of insulation near its respective distal end surface. The membrane is preferably sized to fit within a catheter of a diameter and length capable of being inserted into the vasculature of a human or animal. Preferably, the stripped cathode has a shorter length than the length of the stripped anode. The elongate anode is preferably supported parallel to the elongate cathode and the anode is folded near the transition between the stripped portion and insulated part. There the anode is folded Into a "U" shape such that the distal end surface thereof faces the distal end surface of the cathode.

The invention further provides a method for manufacturing an electrochemical sensor comprising a cathode and an anode immersed in an electrolyte, characterized in that it comprises the steps:
folding an elongate insulated conductor, having an exposed uninsulated distal portion terminating in an exposed uninsulated end surface and a proximal end, into a "U" shape so that the exposed uninsulated distal portion forms one arm of the "U" and the exposed uninsulated end surface faces in the same direction as the proximal end;
positioning an elongate insulated conductor with an exposed uninsulated distal portion terminating in an exposed uninsulated end surface adjacent the elongate insulated conductor, so that the exposed uninsulated end surfaces of each conductor face each other to form a cathode and a facing anode with a gap therebetween;
placing the positioned cathode and facing anode within and parallel to the axis of a hollow tube defined by a gas-permeable membrane;
sealing the insulated portions of the conductors within the proximal end of the tube;
filling the tube with electrolyte so that the gap between the cathode and the anode and the volume surrounding the cathode and anode is filled with electrolyte; and
sealing the distal end of the tube to form a closed chamber containing the cathode, anode and electrolyte.

The invention will be more particularly described with reference to a preferred embodiment illustrated in the accompanying drawings, in which:-
Figure 1 is a perspective and schematic view of an electrical circuit;
Figure 2 is an enlarged side view in (partial) cross section of a prior art oxygen sensor such as in Figure 1 as would be seen if the cross section were taken along line 2-2 in Figure 1. The sensor in Figure 2 is like that disclosed in United States Patent 3,905,888;
Figure 3 is an enlarged side view in (partial) cross section of an oxygen sensor, such as in Figure 1 as would be seen if the cross section were taken along line 3-3 in Figure 1. The sensor in Figure 3 is an approach to resolve the difficulties of the arrangement in Figure 2; and
Figure 4 is an enlarged side view in (partial) cross section of an oxygen sensor, such as in Figure 1, but as would be seen if the cross section were taken along line 4-4 in Figure 1. The sensor in Figure 4 includes an improved arrangement for the cathode and anode, according to a preferred embodiment of the present invention.

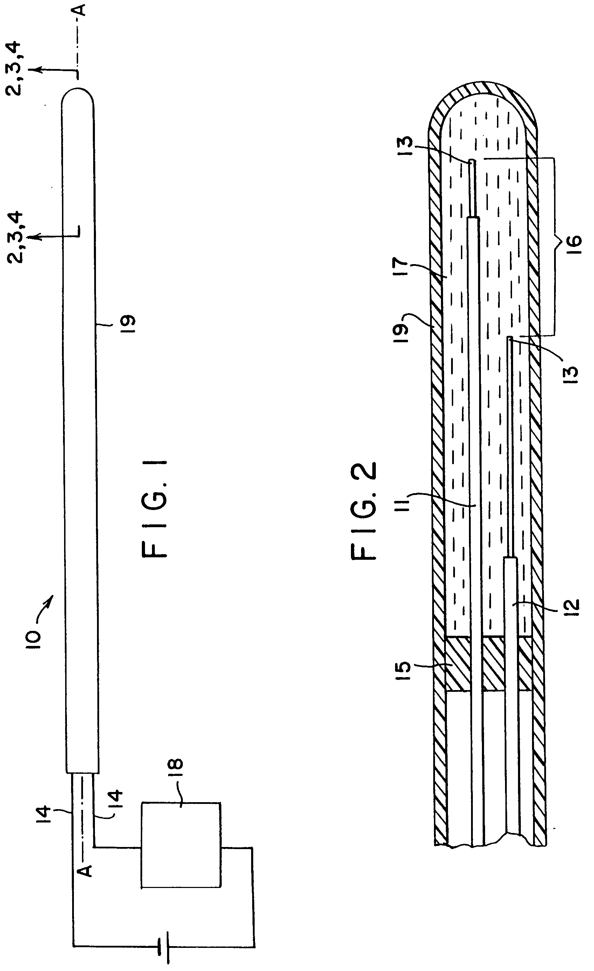

Figure 1 of the drawings illustrates, in schematic form, an electrical circuit 10 comprising a closed chamber defined by a gas permeable membrane 19 which contains two elongate insulated conductors (see Figures 2-4) having proximal ends 14 connected to a current measuring device 18 in circuit with a power source.

The closed chamber of the electrical circuit 10 in a preferred embodiment comprises an electrochemical sensor for placement in the vasculature of a human or animal and so is of a diameter sufficiently small so as to be accommodated within a catheter adapted to be inserted in a blood vessel, such as an artery.

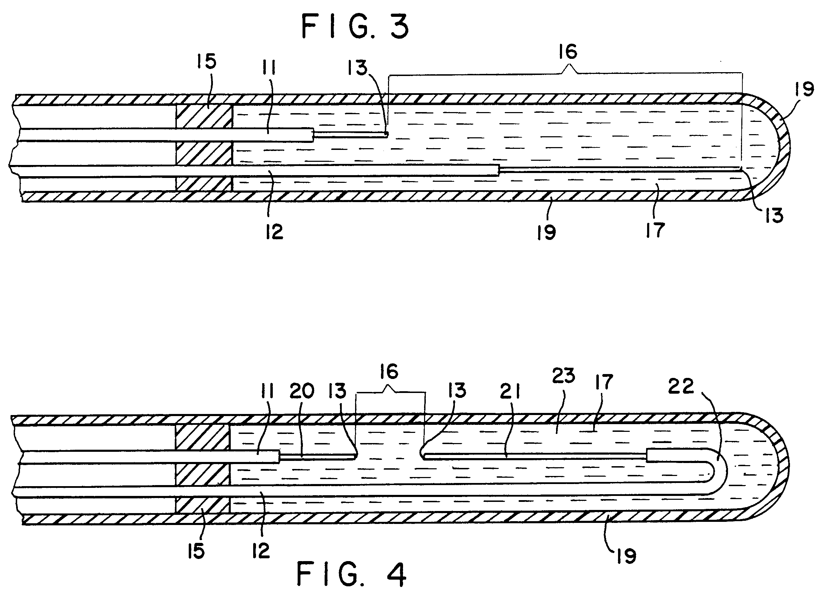

As shown in Figures 2, 3 and 4, the electrochemical sensor comprises a plurality of elongate insulated conductors 11 and 12 each having an exposed uninsulated distal end surface 13 and a proximal end 14 (Figure 1). The conductors, 11 and 12 are, in the preferred embodiment of Figure 4, thin wires made of silver for the cathode and silver or silver chloride for the anode, with polyester insulation. The preferred embodiment is designed to measure the partial pressure of oxygen entrained in the blood stream.

The distal end surface 13 of each conductor 11 or 12 is preferably substantially normal to its elongate insulated conductor wire. A support 15 associated with the plurality of elongate insulated conductors 11 or 12 positions each conductor 11 or 12 with its uninsulated distal end surface 13 in generally facing relation to the distal end surfaces 13 of one or more other elongate insulated conductors 11 or 12.

A gap 16 between the facing distal end surfaces 13 of the elongate insulated conductor wires defines approximately equal distances between the facing distal end surfaces. An electrolyte 17 is disposed within the gap 16 so that the electrochemistry permits flow of electric current across the gap 16 and between the distal end surfaces 13 as a function of the amount of in vivo entrained gas, i.e. oxygen. The electrolyte 17 preferably is a buffered potassium chloride solution. A current sensitive measuring device 18 in circuit with a power source as shown in Figure 1, for example, an ampere meter and a battery, is connected to the proximal ends 14 of the elongate insulated conductors 11 and 12. Specifically, the proximal end of the anode connects to the positive terminal of the battery in a manner well known. The ampere meter thus monitors changes in the flow of current through the electrolyte 17 between the distal end surfaces 13.

The preferred embodiment of the electrical circuit 10 has two conductors 11 and 12 in the plurality. Figures 2, 3, and 4 show only two conductors 11 and 12. The facing distal end surfaces 13 are substantially parallel to each other. Although parallel facing ends are not essential, the ends should be adjacent to one another so that an electrochemical cell therebetween consumes the uninsulated wire thereof only from the distal end toward the proximal end. That was not the case in the known gas sensors.

To separate the cell from the blood stream while permitting measurement of in vivo gas the electrolyte 17 is contained within a compartment 23 defined by an oxygen gas permeable membrane 19 that permits oxygen to diffuse therethrough. In the preferred embodiment of Figure 4, the conductor 11 is an insulated silver wire and the cathode 20 is an exposed distal portion thereof. The conductor 12 is an insulated silver or silver chloride wire and the anode 21 is an exposed distal portion thereof. Each of the cathode and anode terminates in an exposed distal end surface 13. The gas permeable membrane 19 is sized to fit within a catheter (not shown) of a diameter and length capable of being inserted into the vasculature of a human or animal.

An electrochemical cell results from the conductor wires, the electrolyte 17 in the gap 16 and the current sensitive measuring device 18 in circuit with a power source such as a battery between the proximal ends 14. The electrochemical cell is an oxygen sensitive device generating a current flow between one conductor wire, that is the anode 21 and another conductor wire, that is the cathode 20. The current is measurable at the proximal ends 14 which are conveniently outside the vasculature.

The preferred oxygen sensor is preferably carried in a protective sheath (not shown) having an overall diameter suitable for insertion through a catheter, for example, of 20 gauge, that is adapted to be inserted into a port of the vasculature of a human or animal. In Figure 4, the stripped cathode 20 has a shorter length than the length of the stripped anode 21. As shown in Figure 4 the elongate anode is supported generally parallel to the cathode 20 and near the transition between the stripped portion 21 and the insulated part of the anode is folded into a "U" shape 22 such that the distal end surface 13 thereof faces the distal end surface 13 of the cathode 21. While the figures show the insulation extends along anode 21 beyond the "U" shaped fold 22, that is not required so long as the distal end surface 13 of anode 21 is closer to the distal end surface 13 of cathode 20 as shown in Figure 4.

A method for manufacturing an electrochemical sensor with the anode 21 and the cathode 20 comprises the first step of folding the conductor 12 near the transition between the insulated part and the stripped portion of the anode 21 into a "U" shape 22 so that the uninsulated distal end surface 13 thereof faces in the direction of the proximal end thereof 14. The second step positions the cathode 20 adjacent the insulated part of the conductor 12 with the uninsulated distal end surface 13 of the cathode 20 facing the distal end surface 13 of the anode 21 with a gap 16 therebetween. Figure 4 shows the placement and configuration of the anode 21 and cathode 20 in the preferred embodiment. The next step comprises placing the positioned cathode 20 and the facing anode 21 within a hollow tube defined by the gas permeable membrane 19 with the anode 21 and cathode 20 parallel to axis A, see Figure 1, of the gas permeable membrane tube 19. Sealing at support 15 the insulated parts of the conductor 11 and conductor 12 proximal the gap 16 provides an electrochemical cell chamber 23 defined by the gas permeable membrane tube 19. Sealing may be performed in any conventional manner known in the art or, preferably, by melting the gas permeable membrane tube 19 to surround and support the conductors 11 and 12 in spaced parallel relation. The chamber 23 is then filled with electrolyte 17 to surround the facing distal end surfaces 13. Sealing the distal end of the gas permeable membrane tube 19 completes manufacture of the electrochemical cell.

## Claims

1. An electrochemical sensor for the determination of the partial pressure of oxygen in a blood stream comprising a cathode and an anode immersed in an electrolyte 17 contained in a chamber 23 defined by an oxygen gas permeable membrane 19, characterized in that each of the cathode 20 and anode 21 comprises the exposed uninsulated distal portion of a plurality of elongate insulated conductors 11, 12 each having an exposed uninsulated distal end surface 13 and a proximal end 14; the elongate insulated conductors are associated with a support 15 which positions each conductor with the insulated portions in parallel and the elongate conductor or conductors 12 which form the anode 21 is folded into a "U" shape 22 so that the exposed distal end surface 13 thereof faces the distal end surface 13 of the cathode 20; the gap 16 between the facing distal end surfaces 13 of the cathode 20 and anode 21 being filled with an electrolyte 17 to permit the flow of electric current energy across the gap; and the proximal end 14 of each of the conductors is adapted to be connected to a current sensitive measuring device 18 in circuit with a power source for monitoring changes in the flow of electric current between the distal end surfaces 13.

2. A sensor according claim 1, characterized in that each of the cathode 20 and anode 21 comprises a single conductor.

3. A sensor according to claim 1 or 2, characterized in that the cathode 20 is made from silver and the anode 21 is made from silver or silver chloride.

4. A sensor according to any one of claims 1 to 3, characterized in that the electrolyte is a buffered potassium chloride solution.

5. An electrical circuit for the in vivo monitoring of the partial pressure of oxygen in a patient's blood stream characterized in that it comprises, in combination, an electrochemical sensor according to any one of claims 1 to 4 connected, through the proximal end 14 of each of the conductors 11, 12, to a current sensitive measuring device 18 in circuit with a power source, whereby changes in the flow of electric current between the distal end surfaces 13 of the conductors may be monitored.

6. A method for manufacturing an electrochemical sensor comprising a cathode 20 and an anode 21 immersed in an electrolyte 17, characterized in that it comprises the steps:
folding an elongate insulated conductor 12, having an exposed uninsulated distal portion 21 terminating in an exposed uninsulated end surface 13 and a proximal end 14, into a "U" shape 22 so that the exposed uninsulated distal portion 21 forms one arm of the "U" and the exposed uninsulated end surface 13 faces in the same direction as the proximal end 14;
positioning an elongate insulated conductor 11 with an exposed uninsulated distal portion 20 terminating in an exposed uninsulated end surface 13 adjacent the elongate insulated conductor 12, so that the exposed uninsulated end surfaces of each conductor face each other to form a cathode 20 and a facing anode 21 with a gap 16 therebetween;
placing the positioned cathode 20 and facing anode 21 within and parallel to the axis of a hollow tube defined by a gas-permeable membrane 19;
sealing the insulated portions of the conductors 11, 12 within the proximal end of the tube;
filling the tube with electrolyte so that the gap 16 between the cathode and the anode and the volume surrounding the cathode and anode is filled with electrolyte; and
sealing the distal end of the tube to form a closed chamber 23 containing the cathode, anode and electrolyte.
